# EUROPÄISCHE PATENTSCHRIFT

(11) **EP 1 789 405 B1**
(45) Veröffentlichungstag und Bekanntmachung des Hinweises auf die Patenterteilung: **17.07.2013**
(21) Anmeldenummer: 05737938.0
(22) Anmeldetag: 11.05.2005
(51) Int. Cl.: C07D 265/36, C07D 413/04, A61K 31/536, A61P 11/00

(54) **NEUE ENANTIOMERENREINE BETAAGONISTEN, VERFAHREN ZU DEREN HERSTELLUNG UND DEREN VERWENDUNG ALS ARZNEIMITTEL**
NOVEL ENANTIOMERICALLY PURE BETA-AGONISTS, METHOD FOR THE PRODUCTION AND THE USE THEREOF IN THE FORM OF A DRUG
NOUVEAUX BETA-AGONISTES ENANTIOMERIQUEMENT PURS, LEUR PROCEDE DE PRODUCTION ET LEUR UTILISATION COMME MEDICAMENT

(30) Priorität: 14.05.2004 DE 102004024454
(43) Veröffentlichungstag der Anmeldung: 30.05.2007
(73) Patentinhaber: BOEHRINGER INGELHEIM INTERNATIONAL GMBH, 55216 Ingelheim (DE); Boehringer Ingelheim Pharma GmbH & Co. KG, 55216 Ingelheim (DE)
(72) Erfinder: KONETZKI, Ingo, 88447 Warthausen (DE); LUSTENBERGER, Philipp, 4056 Basel (SZ); SIEGER, Peter, 88441 Mittelbiberach (DE)
(74) Vertreter: Simon, Elke Anna Maria
(86) Internationale Anmeldenummer: PCT/EP2005/005079
(87) Internationale Veröffentlichungsnummer: WO 2005/111005

(56) Entgegenhaltungen:
- EP-A- 0 073 505
- EP-A- 0 321 864
- WO-A-2004/045618
- WO-A-2005/110990
- US-A- 4 460 581
- US-A- 4 656 168
- HAMADA T ET AL: "Practical Synthesis of Optically Active Styrene Oxides via Reductive Transformation of 2-Chloroacetophenones with Chiral Rhodium Catalysts" ORGANIC LETTERS, Bd. 4, Nr. 24, 2002, Seiten 4373-4376, XP002337267 AMERICAN CHEMICAL SOCIETY in der Anmeldung erwähnt

## Beschreibung

Die vorliegende Erfindung betrifft enantiomerenreine Verbindungen der allgemeinen Formel **1** 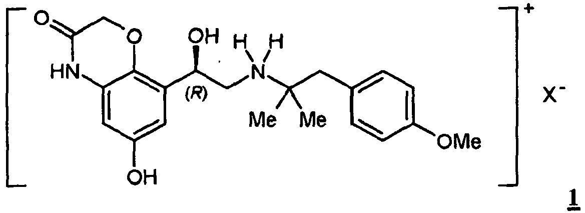 worin die Verbindungen in einer Enantiomerenreinheit von wenigstens 85%ee vorliegen und X⁻ Chlorid bedeutet, Verfahren zu deren Herstellung und deren Verwendung als Arzneimittel, insbesondere als Arzneimittel für die Behandlung von Atemwegserkrankungen

### Hintergrund der Erfindung

Betamimetika (ß-adrenerge Substanzen) sind aus dem Stand der Technik bekannt. Beispielsweise sei diesbezüglich auf die Offenbarung der US 4,460,581 verwiesen, die Betamimetika zur Therapie unterschiedlichster Erkrankungen vorschlägt.

Zur medikamentösen Therapie von Erkrankungen ist es häufig wünschenswert, Arzneimittel mit einer längeren Wirkungsdauer bereitzustellen. Hierdurch kann in der Regel gewährleistet werden, daß die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei.

Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, daß der Patient sich relativ schnell an die regelmäßige Einnahme des Medikaments zu bestimmten Tageszeiten gewöhnen kann.

Es ist Aufgabe der vorliegenden Erfindung, Betamimetika bereitzustellen, die einerseits bei der Therapie von Atemwegserkrankungen einen therapeutischen Nutzen entfalten und darüberhinaus durch eine längere Wirkdauer gekennzeichnet sind und somit zur Herstellung von Arzneimitteln mit längerer Wirksamkeit Verwendung finden können. Es ist insbesondere Aufgabe der vorliegenden Erfindung, Betamimetika bereitzustellen, die aufgrund ihrer langen Wirksamkeit zur Herstellung eines zur Therapie von Atemwegserkrankungen einmal täglich applizierbaren Arzneimittels eingesetzt werden können. Neben den vorstehend genannten Aufgaben ist es ferner Ziel der vorliegenden Erfindung, solche Betamimetika bereitzustellen, die nicht nur außerordentlich potent, sondern ferner durch ein hohes Maß an Selektivität gegenüber dem β₂-Adrenozeptor gekennzeichnet sind. Darüberhinaus ist es Aufgabe der vorliegenden Erfindung, Betamimetika bereitzustellen, die aufgrund ihrer physicochemischen Eigenschaften in besonderer Art und Weise zur Herstellung von für die inhalative Applikation besonders geeigneten Arzneimittelformulierungen verwendbar sind. Insbesondere ist es Aufgabe der vorliegenden Erfindung, Betamimetika bereitzustellen, die neben den vorstehend genannten Eigenschaften eine besondere Eignung zur Herstellung von Inhalationspulvern und Suspensionsaerosolen aufweisen.

### Detaillierte Beschreibung der Erfindung

Überraschenderweise wurde gefunden, daß die vorstehend genannten Aufgaben durch Verbindungen der allgemeinen Formel **1** gelöst werden.

Die vorliegende Erfindung betrifft enantiomerenreine Verbindungen der allgemeinen Formel **1** 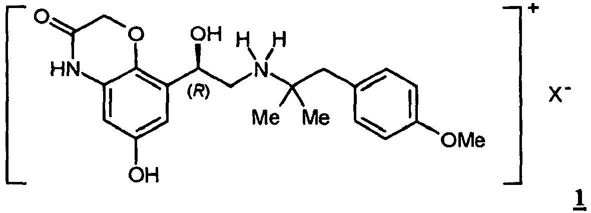 worin die Verbindungen in einer Enantiomerenreinheit von wenigstens 85%ee vorliegen und X⁻ Chlorid bedeutet, gegebenenfalls in Form eines Tautomers, Mischungen der Tautomere, Hydrate oder Solvate.

Besonders bevorzugt sind ferner enantiomerenreine Verbindungen der Formel **1** in kristalliner Form, gegebenenfalls in Form ihrer kristallinen Tautomere, kristallinen Hydrate oder kristallinen Solvate. Besonders bevorzugt sind hierbei enantiomerenreine, kristalline Verbindungen der Formel **1**, gegebenenfalls in Form Ihrer kristallinen Tautomere, kristallinen Hydrate oder kristallinen Solvate, die ferner dadurch gekennzeichnet sind, dass es sich um kristalline Verbindungen handelt, die lediglich in einer einzigen Kristallmodifikationen vorliegen.

Unter der Bezeichnung eine einzige Kristallmodifikation werden dabei kristalline Verbindungen der Formel **1** verstanden, die nicht ein Gemisch gegebenenfalls existierender polymorpher Kristallmodifikationen darstellen.

Die erfindungsgemäßen Verbindungen der Formel **1** zeichnen sich durch vielfältige Anwendungsmöglichkeiten auf therapeutischem Gebiet aus. Hervorzuheben sind erfindungsgemäß solche Anwendungsmöglichkeiten, für welche die erfindungsgemäßen Verbindungen der Formel **1** aufgrund ihrer pharmazeutischen Wirksamkeit als Betamimetikum bevorzugt zur Anwendung gelangen können.

Ein weiterer Aspekt der vorliegenden Erfindung betrifft dementsprechend die vorstehend genannten enantiomerenreinen Verbindungen der Formel **1** als Arzneimittel. Die vorliegende Erfindung betrifft ferner die Verwendung der vorstehend genannten Verbindungen der Formel **1** zur Herstellung eines Arzneimittels zur Behandlung von Atemwegserkrankungen.

Die vorliegende Erfindung betrifft bevorzugt die Verwendung der vorstehend genannten Verbindungen der Formel **1** zur Herstellung eines Arzneimittels zur Behandlung von Atemwegserkrankungen, die ausgewählt sind aus der Gruppe bestehend aus Obstruktive Lungenerkrankungen unterschiedlicher Genese, Lungenemphyseme unterschiedlicher Genese, Restriktive Lungenerkrankungen, Interstitielle Lungenerkrankungen, Zystische Fibrose, Bronchitiden unterschiedlicher Genese, Bronchiektasen, ARDS (adult respiratory distress syndrom) und alle Formen des Lungenödems.

Bevorzugt ist die Verwendung von Verbindungen der Formel **1** zur Herstellung eines Arzneimittels zur Behandlung von Obstruktive Lungenerkrankungen die ausgewählt sind aus der Gruppe bestehend aus COPD (chronisch obstruktive pulmonale Erkrankung), Asthma Bronchiale, pädiatrisches Asthma, schweres Asthma, akuter Asthma-Anfall und chronische Bronchitis, wobei die Verwendung zur Herstellung eines Arzneimittels zur Behandlung von Asthma Bronchiale erfindungsgemäß besonders bevorzugt ist.

Bevorzugt ist ferner die Verwendung von Verbindungen der Formel **1** zur Herstellung eines Arzneimittels zur Behandlung von Lungenemphysemen die ihren Ursprung haben in COPD (chronisch obstruktive pulmonale Erkrankung) oder α1-Proteinase-Inhibitor-Mangel.

Bevorzugt ist ferner die Verwendung von Verbindungen der Formel **1** zur Herstellung eines Arzneimittels zur Behandlung von Restriktiven Lungenerkrankungen, die ausgewählt sind aus der Gruppe bestehend aus Allergische Alveolitis, durch berufliche Noxen ausgelöste restriktive Lungenerkrankungen wie Asbestose oder Silikose und Restriktion aufgrund von Lungentumoren, wie beispielsweise Lymphangiosis carcinomatosa, bronchoalveoläres Karzinom und Lymphome.

Bevorzugt ist ferner die Verwendung von Verbindungen der Formel **1** zur Herstellung eines Arzneimittels zur Behandlung von Interstitiellen Lungenerkrankungen, die ausgewählt sind aus der Gruppe bestehend aus infektiös bedingte Pneumonien, wie beispislweise aufgrund einer Infektion mit Viren, Bakterien, Pilzen, Protozoen, Helminthen oder anderen Erregern, Pneumonitis aufgrund unterschiedlicher Genese, wie beispielsweise Aspiration und Linksherzinsuffizienz, Strahlen-induzierte Pneumonitis oder Fibrose, Kollagenosen, wie beispielsweise Lupus erythematodes, sytemische Sklerodermie oder Sarkoidose, Granulomatosen, wie beispielsweise Morbus Boeck, idiopathische interstitielle Pneumonie oder idiopathische pulmonäre Fibrose (IPF).

Bevorzugt ist ferner die Verwendung von Verbindungen der Formel **1** zur Herstellung eines Arzneimittels zur Behandlung von Zystischer Fibrose bzw. Mukoviszidose.

Bevorzugt ist ferner die Verwendung von Verbindungen der Formel **1** zur Herstellung eines Arzneimittels zur Behandlung von Bronchitiden, wie beispielsweise Bronchitis aufgrund bakterieller oder viraler Infektion, Allergische Bronchitis und Toxische Bronchitis.

Bevorzugt ist ferner die Verwendung von Verbindungen der Formel **1** zur Herstellung eines Arzneimittels zur Behandlung von Bronchiektasen.

Bevorzugt ist ferner die Verwendung von Verbindungen der Formel **1** zur Herstellung eines Arzneimittels zur Behandlung von ARDS (adult respiratory distress syndrom).

Bevorzugt ist ferner die Verwendung von Verbindungen der Formel **1** zur Herstellung eines Arzneimittels zur Behandlung von Lungenödemen, beispielsweise toxischer Lungenödeme nach Aspiration oder Inhalation von toxischen Substanzen und Fremdstoffen.

Besonders bevorzugt betrifft die vorliegende Erfindung die Verwendung der Verbindungen der Formel **1** zur Herstellung eines Arzneimittels zur Behandlung von Asthma oder COPD. Von besonderer Bedeutung ist ferner die vorstehend genannte Verwendung von Verbindungen der Formel **1** zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Ferner betrifft die vorliegende Erfindung ein Verfahren zur Behandlung der vorstehend genannten Erkrankungen, dadurch gekennzeichnet, daß eine oder mehrere der vorstehend genannten Verbindungen der Formel **1** in therapeutisch wirksamen Mengen appliziert werden. Die vorliegende Erfindung betrifft bevorzugt Verfahren zur Behandlung von Asthma oder COPD, dadurch gekennzeichnet, daß eine oder mehrere der vorstehend genannten Verbindungen der Formel **1** in therapeutisch wirksamen Mengen einmal täglich appliziert werden.

Als Alkylgruppen werden, soweit nicht anders angegeben, verzweigte und unverzweigte Alkylgruppen mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Die Bezeichnung enantiomerenrein beschreibt im Rahmen der vorliegenden Erfindung Verbindungen der Formel **1**, die in einer Enantiomerenreinheit von wenigstens 85%ee, bevorzugt von wenigstens 90%ee, besonders bevorzugt von ≥ 95%ee vorliegen. Die Bezeichnung ee (enantiomeric excess) ist im Stand der Technik bekannt und beschreibt den optischen Reinheitsgrad chiraler Verbindungen.

Die Herstellung der erfindungsgemäßen Verbindungen kann gemäß der in Schema 1 skizzierten Vorgehensweise erfolgen.

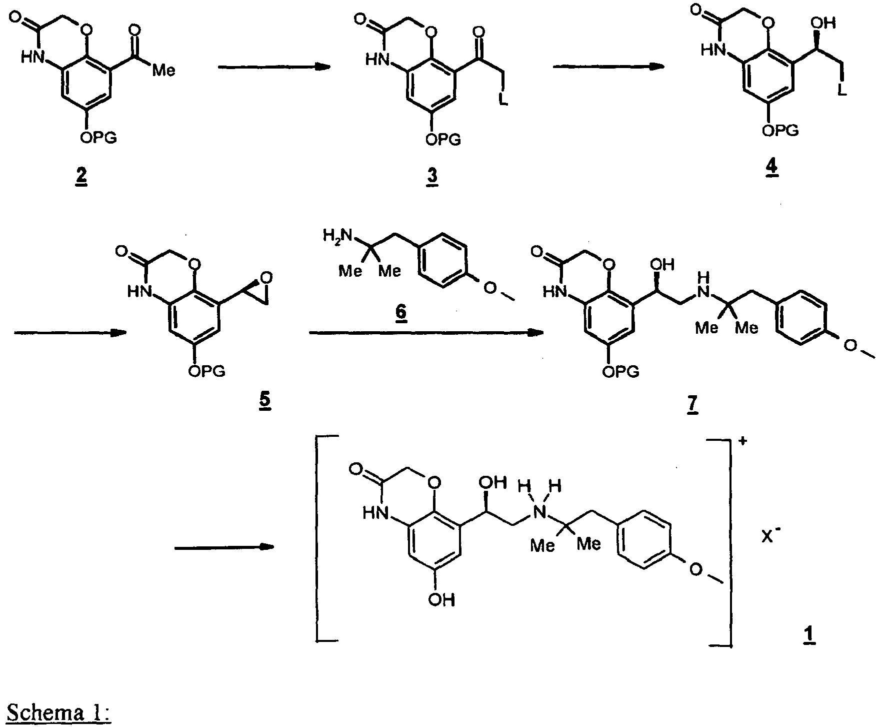

In den in Schema 1 genannten Verbindungen der Formeln **2** bis **5** und **7** steht die Gruppe OPG für eine durch eine Schutzgruppe (PG) geschützte Hydroxylfunktion. Zur Auswahl geeigneter Schutzgruppen für die Hydroxylgruppe sei dabei auf den Stand der Technik verwiesen, wie er beispielsweise in Protective Groups in Organic Synthesis, T.W. Greene und P.G.M. Wuts, John Wiley & Sons Inc, third Edition, 1999 dargelegt ist.

Bevorzugt steht OPG für eine Gruppe, die ausgewählt ist aus der Gruppe bestehend aus -O-C₁-C₄-Alkyl, -O-Benzyl oder -O-CO-C₁-C₄-Alkyl, bevorzugt -O-Methyl, -O-Benzyl oder -O-Acetyl, besonders bevorzugt -O-Methyl oder -O-Benzyl, besonders bevorzugt -O-Benzyl.

In den in Schema 1 genannten Verbindungen der Formeln **3** und **4** steht die Gruppe L für eine Abgangsgruppe. Bevorzugt steht L für eine Abgangsgruppe, die ausgewählt ist aus der Gruppe bestehend aus Chlor, Brom, Iod, Methansulfonat, Trifluormethansulfonat und p-Toluolsulfonat, bevorzugt Chlor oder Brom, besonders bevorzugt Chlor.

Ausgehend von 8-Acetyl-6-benzyloxy-4H-benzo[1,4]oxazin-3-on (**2**) erfolgt die Darstellung der Verbindungen der Formel **3** in aus dem Stand der Technik bekannter Art und Weise Die Verbindung der Formel **3** wird dann in Gegenwart eines chiralen Übergangsmetallkatalysators enantioselektiv in den chiralen Alkohol der Formel **4** überführt, der dann unter geeigneten Bedingungen zum chiralen Oxiran der Formel **5** reagiert. Verfahren zur Durchführung der enantioselektiven Synthese von Oxiranen sind im Stand der Technik bekannt (siehe beispielsweise Hamada et al., Org. Letters 2002, 4, 4373-4376).

Durch Umsetzung der Oxirane **5** mit den Aminen der Formel **6** werden die Verbindungen der Formel **7** erhalten, die nach Abspaltung der Schutzgruppe (PG) in die Salze der Formel **1** überführt werden können.

Die enantiomerenreinen Verbindungen der Formel **1** können gegebenenfalls auch gemäß der in Schema 2 dargestellten Vorgehensweise erhalten werden.

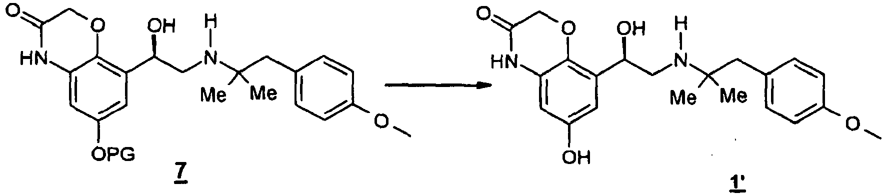

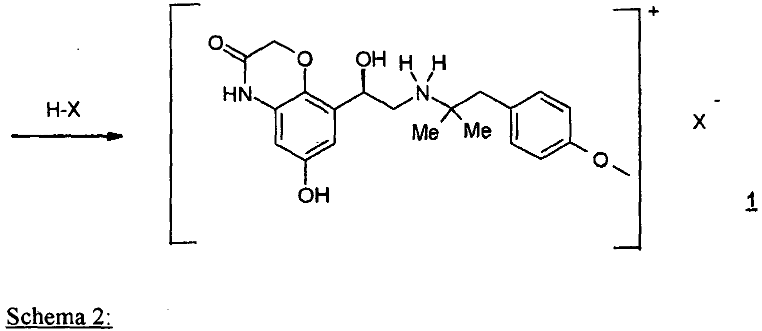

Die gemäß Schema 1 erhältliche Verbindung der Formel **7** kann danach gegebenenfalls zunächst in die freien Basen der Formel **1'** überführt werden. Die freie Base der Formel **1'** wird durch Umsetzung mit einer geeigneten Säure H-X in die Verbindungen der Formel **1** überführt, die durch Fällung in einem geeigneten Lösemittel, beispielsweise in einem Alkohol, bevorzugt in einem Alkohol ausgewählt aus Isopropanol, Ethanol oder Methanol, gegebenenfalls Mischungen davon, wie beispielsweise Isopropanol/Methanol-Gemische, in kristalliner Form erhältlich sind.

Die durchgerührten Umsetzungen sind exemplarisch im nachstehenden experimentellen Teil dieser Patentanmeldung erläutert. Hierbei dienen die nachstehend beschriebenen Synthesebeispiele der weitergehenden Illustration von neuen erfindungsgemäßen Verbindungen. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

### Beispiel 1: 6-Hydroxy-8-{(R)-1-hydroxy-2-[2-(4-methoxy-phenyl)-1,1-dimethyl-ethylamino]-ethyl}-4H-benzo[1,4]oxazin-3-on-Hydrochlorid

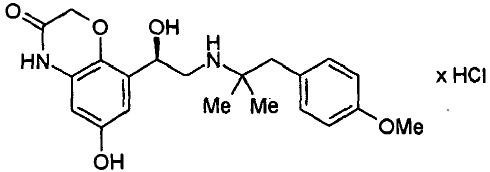

### a) 1-(5-Benzyloxy-2-hydroxy-3-nitro-phenyl)-ethanon

Zu einer Lösung von 81.5 g (0.34 mol) 1-(5-Benzyloxy-2-hydroxy-phenyl)-ethanon in 700 mL Essigsäure werden unter Kühlung mit dem Eisbad 18 mL rauchende Salpetersäure so zugetropft, dass die Temperatur nicht über 20°C steigt. Anschließend wird die Reaktionsmischung zwei Stunden bei Raumtemperatur gerührt, auf Eiswasser gegossen und filtriert. Das Produkt wird in Isopropanol umkristallisiert, abgesaugt und mit Isopropanol und Diisopropylether gewaschen. Ausbeute: 69.6 g (72%); Massenspektroskopie [M+H]⁺ = 288.

### b) 1-(3-Amino-5-benzyloxy-2-hydroxy-phenyl)-ethanon

69.5 g (242 mmol) 1-(5-Benzyloxy-2-hydroxy-3-nitro-phenyl)-ethanon werden in 1.4 L Methanol gelöst und in Gegenwart von 14 g Rhodium auf Kohle (10%ig) als Katalysator bei 3 bar und Raumtemperatur hydriert. Anschließend wird der Katalysator abfiltriert und das Filtrat eingeengt. Der Rückstand wird ohne zusätzliche Aufreinigung weiter umgesetzt. Ausbeute: 60.0 g (96%), R_{f}-Wert = 0.45 (Dichlormethan auf Kieselgel).

### c) 8-Acetyl-6-benzyloxy-4H-benzo[1,4]oxazin-3-on

Zu 60.0 g (233 mmol) 1-(3-Amino-5-benzyloxy-2-hydroxy-phenyl)-ethanon und 70.0 g (506 mmol) Kaliumcarbonat werden unter Kühlung mit dem Eisbad 21.0 mL (258 mmol) Chloracetylchlorid getropft. Anschließend wird über Nacht bei Raumtemperatur und dann 6 Stunden unter Rückfluß gerührt. Die heiße Reaktionsmischung wird filtriert, dann auf ca. 400 mL eingeengt und mit Eiswasser versetzt. Der ausfallende Niederschlag wird abgesaugt, getrocknet und durch Chromatographie an einer kurzen Kieselgelsäule (Dichlormethan:Methanol = 99:1) gereinigt. Die produkthaltigen Fraktionen werden eingeengt, in Isopropanol/Diisopropylether suspendiert, abgesaugt und mit Diisopropylether gewaschen. Ausbeute: 34.6 g (50%); Massenspektroskopie [M+H]⁺ = 298.

### d) 6-Benzyloxy-8-(2-chlor-acetyl)-4H-benzo[1,4]oxazin-3-on

13.8 g (46.0 mmol) 8-Acetyl-6-benzyloxy-4H-benzo[1,4]oxazin-3-on und 35.3 g (101.5 mmol) Benzyltrimethylammonium-dichloriodat werden in 250 mL Dichlorethan, 84 mL Eisessig und 14 mL Wasser 5 Stunden bei 65°C gerührt. Nach Abkühlung auf Raumtemperatur wird mit 5%iger Natriumhydrogensulfit-Lösung versetzt und 30 Minuten gerührt. Der ausgefallene Feststoff wird abgesaugt, mit Wasser und Diethylether gewaschen und getrocknet. Ausbeute: 13.2 g (86%); Massenspektroskopie [M+H]⁺ = 330/32.

### e) 6-Benzyloxy-8-((R)-2-chlor-1-hydroxy-ethyl)-4H-benzo[1,4]-oxazin-3-on

Die Durchführung erfolgt in Analogie zu einem in der Literatur beschriebenen Verfahren (Org. Lett. 2002, 4, 4373-4376).

Zu 13.15 g (39.6 mmol) 6-Benzyloxy-8-(2-chlor-acetyl)-4H-benzo[1,4]oxazin-3-on und 25.5 mg (0.04 mmol) Cp*RhCl[(S,S)-TsDPEN] (Cp* = Pentamethylcyclopentadienyl und TsDPEN = (1S,2S)-N-p-Toluensulfonyl-1,2-diphenylethylendiamin) in 40 mL Dimethylformamid werden bei -15°C 8 mL eines Gemisches aus Ameisensäure und Triethylamin (Molverhältnis = 5:2) getropft. Man lässt 5 Stunden bei dieser Temperatur Rühren, gibt dann 25 mg Katalysator nach und rührt über Nacht bei -15°C. Die Reaktionsmischung wird mit Eiswasser versetzt und filtriert. Der Filterrückstand wird in Dichlormethan gelöst, mit Natriumsulfat getrocknet und vom Lösungsmittel befreit. Der Rückstand wird chromatographiert (Dichlormethan/Methanol-Gradient) und das Produkt in Diethylether/Diisopropylether umkristallisiert. Ausbeute: 10.08 g (76%); R_{f}-Wert = 0.28 (Dichlormethan:Methanol = 50:1 auf Kieselgel).

### f) 6-Benzyloxy-8-(R)-oxiranyl-4H-benzo[1,4]oxazin-3-on

10.06 g (30.1 mmol) 6-Benzyloxy-8-((R)-2-chlor-1-hydroxy-ethyl)-4H-benzo[1,4]-oxazin-3-on werden in 200 mL Dimethylformamid gelöst. Die Lösung wird bei 0°C mit 40 mL einer 2 molaren Natronlauge versetzt und bei dieser Temperatur 4 Stunden gerührt. Die Reaktionsmischung wird auf Eiswasser gegossen, 15 Minuten gerührt und dann filtriert. Der Feststoff wird mit Wasser gewaschen und getrocknet. Ausbeute: 8.60 g (96%); Massenspektroskopie [M+H]⁺ = 298.

### g) 6-Benyloxy-8-{(R)-1-hydroxy-2-[2-(4-methoxy-phenyl)-1,1-dimethyl-ethylamino]-ethyl}-4H-benzo[1,4,]oxazin-3-on

5.25 g (17.7 mmol) 6-Benzyloxy-8-(R)-oxiranyl-4H-benzo[1,4]oxazin-3-on und 6.30 g (35.1 mmol) 2-(4-Methoxy-phenyl)-1,1-dimethyl-ethylamin werden mit 21 mL Isopropanol versetzt und 30 Minuten bei 135°C unter Mikrowelleneinstrahlung in einem geschlossenen Reaktionsgefäß gerührt. Das Lösungsmittel wird abdestilliert und der Rückstand chromatographiert (Aluminiumoxid; Ethylacetat/Methanol-Gradient). Das so erhaltene Produkt wird durch Umkristallisation aus einem Diethylether/Diisopropylether-Gemisch weiter gereinigt. Ausbeute: 5.33 g (63%); Massenspektroskopie [M+H]⁺ = 477.

### h) 6-Hydroxy-8-{(R)-1-hydroxy-2-[2-(4-methoxy-phenyl)-1,1-dimethyl-ethylamino]-ethyl}-4H-benzo[1,4]oxazin-3-on-Hydrochlorid

Eine Suspension von 5.33 g (11.2 mmol) 6-Benyloxy-8-{(R)-1-hydroxy-2-[2-(4-methoxyphenyl)-1,1-dimethyl-ethylamino]-ethyl}-4H-benzo[1,4,]oxazin-3-on in 120 mL Methanol wird mit 0.8 g Palladium auf Kohle (10%ig) versetzt, auf 50°C erwärmt und bei 3 bar Wasserstoffdruck hydriert. Anschließend wird der Katalysator abgesaugt und das Filtrat eingeengt. Man löst den Rückstand in 20 mL Isopropanol und gibt 2.5 mL 5 molare Salzsäure in Isopropanol zu. Das Produkt wird mit 200 mL Diethylether ausgefällt, abgesaugt und getrocknet. Ausbeute: 4.50 g (95%, Hydrochlorid); Massenspektroskopie [M+H]⁺ = 387.

In analoger Art und Weise werden durch Umsetzung der Verbindung 6-Benzyloxy-8-(R)-oxiranyl-4H-benzo[1,4]oxazin-3-on (Beispiel 1, Stufe f) mit dem entsprechenden Amin die folgenden Verbindungen der Formel **1** erhalten.

Sofern die Verbindungen der Formel **1** gemäß der vorstehend exemplarisch angegebenen synthetischen Vorgehensweise gegebenenfalls nicht zu einheitlichen Kristallmodifikationen führen, kann es dienlich sein, die erhaltenen Salze der Formel **1** in geeigneten Lösemitteln auszukristallisieren. Darüber hinaus sind aus den vorstehend genannten Beispielen andere Salze durch Anwendung an und für sich im Stand der Technik bekannter Methoden zugänglich.

### Beispiel 2: 6-Hydroxy-8-{1-hydroxy-2-[2-(4-methoxy-phenyl)-1,1-dimethyl-ethylamino]-ethyl}-4H-benzo[1,4,]oxazin-3-on (freie Base)

500 mg (1.2 mmol) 6-Hydroxy-8-{(R)-1-hydroxy-2-[2-(4-methoxy-phenyl)-1,1-dimethyl-ethylamino]-ethyl}-4H-benzo[1,4,]oxazin-3-on hydrochlorid werden zunächst mit Ethylacetat versetzt. Die organische Phase wird mit wässriger Kaliumcarbonat-Lösung ausgeschüttelt, mit Natriumsulfat getrocknet und vom Lösungsmittel befreit. Die so erhaltene freie Base wird in Acetonitril unter Zugabe von wenigen Tropfen Wasser gelöst. Der ausfallende Feststoff wird abgesaugt, gewaschen und getrocknet. Ausbeute: 168 mg (37%); Massenspektroskopie [M+H]⁺ = 387; Schmelzpunkt = 128°C.

Das hochkristalline Produkt wurde mit Hilfe der Röntgenpulverbeugung weitergehend untersucht. Zur Aufnahme des Röntgenpulverdiagramms wurde wie folgt verfahren.

Das Röntgenpulverdiagramm wurde im Rahmen der vorliegenden Erfindung aufgenommen mittels eines Bruker D8 Advanced mit einem OED (= ortsempfindlicher Detektor) (CuK_{α} - Strahlung, λ = 1.5418 Å, 30 kV, 40 mA).

Für die hochkristalline Verbindung wurden u.a. die folgenden charakteristischen Werte dₕₖₗ [Å], die die bestimmten Gitterebenenabstände in Å angeben, bestimmt:
d= 14,96 Å; 9,63 Å; 7,05 Å; 5,57 Å; 5,28 Å; 5,05 Å; 4,63 Å und 3,73 Å.

### Beispiel 3: 6-Hydroxy-8-{(R)-1-hydroxy-2-[2-(4-methoxy-phenyl)-1,1-dimethyl-ethylamino]-ethyl}-4H-benzo[1,4,]oxazin-3-on-hydrochlorid

300 mg (0.71 mmol) 6-Hydroxy-8-{(R)-1-hydroxy-2-[2-(4-methoxy-phenyl)-1,1-dimethyl-ethylamino]-ethyl}-4H-benzo[1,4,]oxazin-3-on hydrochlorid werden durch Erwärmen in 4 mL Isopropanol gelöst. Die Lösung wird auf Raumtemperatur abgekühlt und dann für 15 Minuten in ein Eisbad gestellt. Der ausgefallene Feststoff wird abgesaugt und getrocknet. Ausbeute: 180 mg (60%); Massenspektroskopie [M+H]⁺ = 387; Schmelzpunkt = 211°C.

Das hochkristalline Produkt wurde mit Hilfe der Röntgenpulverbeugung weitergehend untersucht. Zur Aufnahme des Röntgenpulverdiagramms wurde wie folgt verfahren.

Das Röntgenpulverdiagramm wurde im Rahmen der vorliegenden Erfindung aufgenommen mittels eines Bruker D8 Advanced mit einem OED (= ortsempfindlicher Detektor) (CuK_{α} - Strahlung, λ = 1.5418 Å, 30 kV, 40 mA).

Für die hochkristalline Verbindung wurden u.a. die folgenden charakteristischen Werte dₕₖₗ [Å], die die bestimmten Gitterebenenabstände in Å angeben, bestimmt:
d= 5,92 Å; 5,81 Å; 5,51 Å; 5,10 Å; 4,65 Å; 4,50 Å; 4,15 Å und 4,00 Å.

Die Verbindungen der Formel **1** können allein oder in Kombination mit anderen erfindungsgemäßen Wirkstoffen der Formel **1** zur erfindungsgemäßen Anwendung gelangen. Gegebenenfalls können die Verbindungen der Formel **1** auch in Kombination mit weiteren pharmakologisch aktiven Wirkstoffen eingesetzt werden.

Bevorzugt betrifft die vorliegende Erfindung ferner Arzneimittelkombinationen, die neben einer oder mehrere, bevorzugt einer Verbindung der Formel **1** als weiteren Wirkstoff einen oder mehrere Verbindungen enthalten, die ausgewählt sind aus den Klassen der Anticholinergika, PDEIV-Inhibitoren, Steroide, LTD4-Antagonisten und EGFR-Hemmer.

Anticholinergika kommen dabei bevorzugt in Betracht, wobei Verbindungen, die ausgewählt sind aus Bromiden und Chloriden der Kationen Tiotropium, Oxitropium, Flutropium, Ipratropium, Glycopyrronium und Trospium von besonderem Interesse sind. Von besonderer Bedeutung ist das Tiotropiumbromid, bevorzugt in Form des kristallinen Tiotropiumbromid Monohydrats, welches aus der WO 02/30928 bekannt ist. Wird das Tiotropiumbromid in den erfindungsgemäßen Arzneimittelkombinationen in wasserfreier Form eingesetzt, so gelangt bevorzugt das wasserfreie kristalline Tiotropiumbromid zur Anwendung, welches aus der WO 03/000265 bekannt ist.

In einer ebenfalls bevorzugten Ausführungsform der vorliegenden Erfindung wird als Anticholinergikum die Verbindung 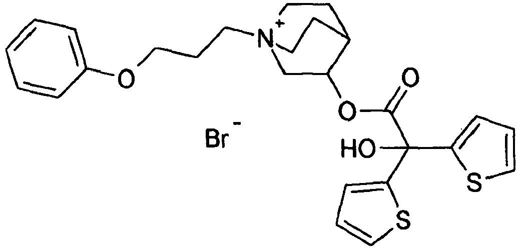 eingesetzt, gegebenenfalls in Form ihrer Enantiomere.

Ferner gelangen als Anticholinergika gegebenenfalls die nachstehenden Verbindungen in Kombination mit den Verbindungen der Formel **1** zum Einsatz:
- 2,2-Diphenylpropionsäuretropenolestermethobromid,
- 2,2-Diphenylpropionsäurescopinestermethobromid,
- 2-Fluor-2,2-Diphenylessigsäurescopinestermethobromid,
- 2-Fluor-2,2-Diphenylessigsäuretropenolestermethobromid,
- 3,3',4,4'-Tetrafluorbenzilsäuretropenolestermethobromid,
- 3,3',4,4'-Tetrafluorbenzilsäurescopinestermethobromid,
- 4,4'-Difluorbenzilsäuretropenolestermethobromid,
- 4,4'-Difluorbenzilsäurescopinestermethobromid,
- 3,3'-Difluorbenzilsäuretropenolestermethobromid,
- 3,3'-Difluorbenzilsäurescopinestermethobromid,
- 9-Hydroxy-fluoren-9-carbonsäuretropenolestermethobromid;
- 9-Fluor-fluoren-9-carbonsäuretropenolestermethobromid;
- 9-Hydroxy-fluoren-9-carbonsäurescopinestermethobromid;
- 9-Fluor-fluoren-9-carbonsäurescopinestermethobromid;
- 9-Methyl-fluoren-9-carbonsäuretropenolestermethobromid;
- 9-Methyl-fluoren-9-carbonsäurescopinestermethobromid;
- Benzilsäurecyclopropyltropinestermethobromid,
- 2,2-Diphenylpropionsäurecyclopropyltropinestermethobromid;
- 9-Hydroxy-xanthen-9-carbonsäurecyclopropyltropinestermethobromid;
- 9-Methyl-fluoren-9-carbonsäurecyclopropyltropinestermethobromid ;
- 9-Methyl-xanthen-9-carbonsäurecyclopropyltropinestermethobromid;
- 9-Hydroxy-fluoren-9-carbonsäurecyclopropyltropinestermethobromid ;
- 4,4'-Difluorbenzilsäuremethylestercyclopropyltropinestermethobromid.
- 9-Hydroxy-xanthen-9-carbonsäuretropenolestermethobromid ;
- 9-Hydroxy-xanthen-9-carbonsäurescopinestermethobromid;
- 9-Methyl-xanthen-9-carbonsäuretropenolestermethobromid ;
- 9-Methyl-xanthen-9-carbonsäurescopinestermethobromid;
- 9-Ethyl-xanthen-9-carbonsäuretropenolestermethobromid;
- 9-Difluormethyl-xanthen-9-carbonsäuretropenolestermethobromid oder ;
- 9-Hydroxymethyl-xanthen-9-carbonsäurescopinestermethobromid.

Werden PDE IV-Inhibitoren in Kombination mit einer oder mehrerer Verbindungen der Formel **1** eingesetzt, sind diese bevorzugt ausgewählt aus der Gruppe bestehend aus Enprofyllin, Theophyllin, Roflumilast, Ariflo (Cilomilast), CP-325,366, BY343, D-4396 (Sch-351591), AWD-12-281 (GW-842470), N-(3,5-Dichloro-1-oxo-pyridin-4-yl)-4-difluoromethoxy-3-cyclopropylmethoxybenzamid, NCS-613, Pumafentine, (-)p-[(4*a*R*, 10*b*S*)-9-Ethoxy-1,2,3,4,4a, 10b-hexahydro-8-methoxy-2-methylbenzo[s][1,6]naphthyridin-6-yl]-N,N-diisopropylbenzamid, (R)-(+)-1-(4-Bromobenzyl)-4-[(3-cyclopentyloxy)-4-methoxyphenyl]-2-pyrrolidon, 3-(Cyclopentyloxy-4-methoxyphenyl)-1-(4-N'-[N-2-cyano-S-methyl-isothioureido]benzyl)-2-pyrrolidon, cis[4-Cyano-4-(3-cyclopentyloxy-4-methoxyphenyl)cyclohexan-1-carbonsäure], 2-carbomethoxy-4-cyano-4-(3-cyclopropylmethoxy-4-difluoromethoxyphenyl)cyclohexan-1-on, cis[4-Cyano-4-(3-cyclopropylmethoxy-4-difluoromethoxyphenyl)cyclohexan-1-ol], (R)-(+)-Ethyl[4-(3-cyclopentyloxy-4-methoxyphenyl)pyrrolidin-2-yliden]acetat, (S)-(-)-Ethyl[4-(3-cyclopentyloxy-4-methoxyphenyl)pyrrolidin-2-yliden]acetat, CDP840, Bay-198004, D-4418, PD-168787, T-440, T-2585, Arofyllin, Atizoram, V-11294A, C1-1018, CDC-801, CDC-3052, D-22888, YM-58997, Z-15370, 9-Cyclopentyl-5,6-dihydro-7-ethyl-3-(2-thienyl)-9*H*-pyrazolo[3,4-c]-1,2,4-triazolo[4,3-a]pyridin und 9-Cyclopentyl-5,6-dihydro-7-ethyl-3-(*tert*-butyl)-9*H*-pyrazolo[3,4-c]-1,2,4-triazolo[4,3-a]pyridin, gegebenenfalls in Form ihrer Racemate, Enantiomere oder Diastereomere und gegebenenfalls in Form ihrer pharmakologisch verträglichen Säureadditionssalze, Solvate und/oder Hydrate. Unter Säureadditionssalzen mit pharmakologisch verträglichen Säuren werden hierbei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Werden Steroide in Kombination mit einer oder mehrerer Verbindungen der Formel 1 eingesetzt, sind diese bevorzugt ausgewählt aus der Gruppe bestehend aus Prednisolon, Prednison, Butixocortpropionat, RPR-106541, Flunisolid, Beclomethason, Triamcinolon, Budesonid, Fluticason, Mometason, Ciclesonid, Rofleponid, ST-126, Dexamethason, 6α, 9α-Difluoro-17α-[(2-furanylcarbonyl)oxy]-11β-hydroxy-16α-methyl-3-oxo-androsta-1,4-dien-17β-carbothionsäure (S)-fluoromethylester, und 6α,9α-Difluoro-11β-hydroxy-16α-methyl-3-oxo-17α-propionyloxy-androsta-1,4-dien-17β-carbothionsäure (S)-(2-oxo-tetrahydro-furan-3S-yl)ester, gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Werden LTD4-Antagonisten in Kombination mit einer oder mehrerer Verbindungen der Formel **1** eingesetzt, sind diese bevorzugt ausgewählt aus der Gruppe bestehend aus Montelukast, 1-(((R)-(3-(2-(6,7-Difluoro-2-quinolinyl)ethenyl)phenyl)-3-(2-(2-hydroxy-2-propyl)phenyl)thio)methylcyclopropan-essigsäure, 1-(((1(R)-3(3-(2-(2,3-Dichlorothieno[3,2-b]pyndin-5-yI)-(E)-ethenyl)phenyl)-3-(2-(1-hydroxy-1-methylethyl)phenyl)propyl)thio)methyl)cyclopropanessigsäure, Pranlukast, Zafirlukast, [2-[[2-(4-tert-Butyl-2-thiazolyl)-5-benzofuranyt]oxymethyl]phenyl]essigsäure, MCC-847 (ZD-3523), MN-001, MEN-91507 (LM-1507), VUF-5078, VUF-K-8707 und L-733321, gegebenenfalls in Form ihrer Racemate, Enantiomere oder Diastereomere, gegebenenfalls in Form ihrer pharmakologisch verträglichen Säureadditionssalze sowie gegebenenfalls in Form ihrer Salze und Derivate, ihrer Solvate und/oder Hydrate. Unter Säureadditionssalzen mit pharmakologisch verträglichen Säuren zu deren Bildung die LTD4-Antagonisten gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden. Unter Salzen oder Derivaten zu deren Bildung die LTD4-Antagonisten gegebenenfalls in der Lage sind, werden beispielsweise verstanden : Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Werden EGFR-Hemmer in Kombination mit einer oder mehrerer Verbindungen der Formel **1** eingesetzt, sind diese bevorzugt ausgewählt aus der Gruppe bestehend aus 4-[(3-Chlor-4-fluorphenyl)amino]-6-{[4-(morpholin-4-yl)-1-oxo-2-buten-1-yl]amino}-7-cyclopropylmethoxy-chinazolin, 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin, 4-[(3-Chlor-4-fluor-phenyl)amino]-6-[2-((S)-6-methyl-2-oxo-morpholin-4-yl)-ethoxy]-7-methoxy-chinazolin, 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin, 4-[(3-Ethinyl-phenyl)amino]-6,7-bis-(2-methoxy-ethoxy)-chinazolin, 4-[(3-Chlor-4-fluorphenyl)amino]-6-{[4-(morpholin-4-yl)-1-oxo-2-buten-1-yl]-amino}-7-[(tetrahydrofuran-2-yl)methoxy]-chinazolin, 4-[(3-Ethinyl-phenyl)amino]-6-{[4-(5,5-dimethyl-2-oxo-morpholin-4-yl)-1-oxo-2-buten-1-yl]amino}-chinazolin, 4-[(3-Chlor-4-fluor-phenyl)amino]-6-(trans-4-methansulfonylamino-cyclohexan-1-yloxy)-7-methoxy-chinazolin, 4-[(3-Chlor-4-fluor-phenyl)amino]-6-(tetrahydropyran-3-yloxy)-7-methoxy-chinazolin, 4-[(3-Chlor-4-fluor-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[2-(2-oxopyrrolidin-1-yl)ethyl]-piperidin-4-yloxy}-7-methoxy-chinazolin, 4-[(3-Ethinyl-phenyl)amino]-6-(1-acetyl-piperidin-4-yloxy)-7-methoxy-chinazolin, 4-[(3-Ethinyl-phenyl)amino]-6-(1-methyl-piperidin-4-yloxy)-7-methoxy-chinazolin, 4-[(3-Ethinyl-phenyl)amino]-6-(1-methansulfonyl-piperidin-4-yloxy)-7-methoxy-chinazolin, 4-[(3-Ethinyl-phenyl)amino]-6-{1-[(morpholin-4-yl)carbonyl]-piperidin-4-yloxy}-7-methoxy-chinazolin, 4-[(3-Chlor-4-fluor-phenyl)amino]-6-{1-[(2-methoxyethyl)carbonyl]-piperidin-4-yloxy}-7-methoxy-chinazolin, 4-[(3-Chlor-4-fluor-phenyl)amino]-6-[cis-4-(N-methansulfonyl-N-methyl-amino)-cyclohexan-1-yloxy]-7-methoxy-chinazolin, 4-[(3-Chlor-4-fluor-phenyl)amino]-6-[cis-4-(N-acetyl-N-methyl-amino)-cyclohexan-1-yloxy]-7-methoxy-chinazolin, 4-[(3-Chlor-4-fluor-phenyl)amino]-6-(trans-4-methylamino-cyclohexan-1-yloxy)-7-methoxy-chinazolin, 4-[(3-Chlor-4-fluor-phenyl)amino]-6-[trans-4-(N-methansulfonyl-N-methyl-amino)-cyclohexan-1-yloxy]-7-methoxy-chinazolin, 4-[(3-Chlor-4-fluor-phenyl)amino]-6-(trans-4-dimethylamino-cyclohexan-1-yloxy)-7-methoxy-chinazolin, 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin, 4-[(3-Chlor-4-fluorphenyl)amino]-6-[2-(2,2-dimethyl-6-oxo-morpholin-4-yl)-ethoxy]-7-[(S)-(tetrahydrofuran-2-yl)methoxy]-chinazolin, 4-[(3-Chlor-4-fluor-phenyl)amino]-6-(1-methansulfonyl-piperidin-4-yloxy)-7-methoxy-chinazolin und 4-[(3-Chlor-4-fluor-phenyl)amino]-6-(1-cyano-piperidin-4-yloxy)-7-methoxy-chinazolin, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.
Unter Säureadditionssalzen mit pharmakologisch verträglichen Säuren zu deren Bildung die vorstehend genannten EGFR-Hemmer gegebenenfalls in der Lage sind, werden beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-p-toluolsulfonat, bevorzugt Hydrochlorid, Hydrobromid, Hydrosulfat, Hydrophosphat, Hydrofumarat und Hydromethansulfonat verstanden.

Geeignete Anwendungsformen zur Applikation der Verbindungen der Formel 1 sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilfslösungsmittel eingesetzt werden können, hergestellt und in Injektionsflaschen oder Ampullen oder Infusionsflaschen abgefüllt.

Die die erfindungsgemäßen Verbindungen der Formel **1** enthaltenden Kapseln können beispielsweise hergestellt werden, indem man die Wirkstoffe mit inerten Trägem, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide), synthetische Gesteinsmehle (z.B. hochdisperse Kieselsäure und Silikate), Zucker (z.B. Rohr-, Milch- und Traubenzucker), Emulgiermittel (z.B. Lignin, Sufitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Bei der erfindungsgemäß besonders bevorzugten Verwendung der Verbindungen der Formel **1** zur Therapie von Atemwegserkrankungen werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfaßt.

Die erfindungsgemäß besonders bevorzugt in kristalliner Form zur Anwendung gelangenden Verbindungen der Formel **1** werden bevorzugt zur Herstellung von Inhalationspulvern eingesetzt. Erfindungsgemäß einsetzbare Inhalationspulver können die kristallinen Verbindungen der Formel **1** entweder allein oder im Gemisch mit geeigneten physiologisch unbedenkliche Hilfsstoffen enthalten.
Sind die Wirkstoffe im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.
Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfststoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt.

Die erfindungsgemäßen Inhalationspulver können mittels aus dem Stand der Technik bekannten Inhalatoren appliziert werden.

Erfindungsgemäße treibgashaltige Inhalationsaerosole können im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendnung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannter Inhalatoren (MDIs = metered dose inhalers) appliziert werden.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel bereits bei Dosen im µg-Bereich durch eine hohe Wirksamkeit aus. Auch oberhalb des µg-Bereichs, lassen sich die Verbindungen der Formel sinnvoll einsetzen. Die Dosierung kann dann beispielsweise auch im Milligrammbereich liegen.

Ein weiterer Aspekt der vorliegenden Erfindung betrifft die vorstehend genannten pharmazeutischen Formulierungen, gekennzeichnet durch einen Gehalt einer Verbindung der Formel 1 als solche, besonders bevorzugt die vorstehend genannten inhalativ applizierbaren pharmazeutischen Formulierungen.

Die nachfolgenden Formulierungsbeipiele illustrieren die vorliegende Erfindung, ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | | |
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| D) | Dosieraerosol | |
|---|---|---|
| | | |
| | Wirkstoff | 0,005 |
| | Sorbitantrioleat | 0,1 |
| | TG134a : TG227 2:1 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| E) | Lösungen (in mg/100ml) | |
|---|---|---|
| | | |
| | Wirkstoff | 333.3 mg |
| | Benzalkoniumchlorid | 10.0 mg |
| | EDTA | 50.0 mg |
| | HCl (ln) | ad pH 3.4 |

Diese Lösung kann in üblicher Art und Weise hergestellt werden.

| F) | Inhalationspulver | |
|---|---|---|
| | Wirkstoff | 12 µg |
| | Lactose Monohydrat | ad 25 mg |

Die Herstellung des Inhalationspulvers erfolgt in üblicher Art und Weise durch Mischen der einzelnen Bestandteile.

## Patentansprüche

1. Enantiomerenreine Verbindungen der Formel 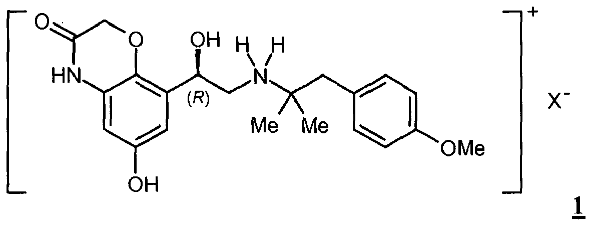 worin die Verbindungen in einer Enantiomerenreinheit von wenigstens 85%ee vorliegen und X⁻ Chlorid bedeutet, gegebenenfalls in Form eines Tautomers, Mischungen der Tautomere, Hydrate oder Solvate.

2. Enantiomerenreine Verbindungen der Formel 1 nach Anspruch 1, **dadurch gekennzeichnet, dass** sie in kristalliner Form vorliegen, gegebenenfalls in Form Ihrer kristallinen Tautomere, kristallinen Hydrate oder kristallinen Solvate.

3. Enantiomerenreine Verbindungen der allgemeinen Formel 1 nach einem der Ansprüche 1 oder 2, dessen mittels eines Bruker D8 Advanced mit einem OED (= ortsempfindlicher Detektor) CuK_{α}, - Strahlung, λ = 1.5418 Å, 30 kV, 40 mA aufgenommenes Röntgenpulverdiagramm folgende Gitterebenenabstände in Å aufweisen: d= 5,92 Å; 5,81 Å; 5,51 Å; 5,10 Å; 4,65 Å; 4,50 Å; 4,15 Å und 4,00 Å.

4. Enantiomerenreine Verbindungen der allgemeinen Formel **1** nach einem der Ansprüche 1 bis 3 als Arzneimittel.

5. Enantiomerenreine Verbindungen der allgemeinen Formel **1** nach einem der Ansprüche 1 bis 3 als Arzneimittel zur Behandlung von Atemwegserkrankungen.

6. Verwendung enantiomerenreiner Verbindungen der allgemeinen Formel **1** nach einem der Ansprüche 1 bis 3 zur Herstellung eines Arzneimittels zur Behandlung von Atemwegserkrankungen.

7. Pharmazeutische Formulierung, **gekennzeichnet durch** einen Gehalt einer Verbindung der Formel **1** gemäß einem der Ansprüche 1 bis 3.

8. Pharmazeutischen Formulierung nach Anspruch 7 , **gekennzeichnet durch** einen Gehalt einer Verbindung der Formel **1** gemäß einem der Ansprüche 1 bis 3 und einer oder mehrerer Verbindungen, die ausgewählt sind aus den Klassen der Anticholinergika, PDEIV-Inhibitoren, Steroide, LTD4-Antagonisten und EGFR-Hemmer.

9. Pharmazeutischen Formulierung nach Anspruch 8, **gekennzeichnet durch** einen Gehalt einer oder mehrerer Anticholinergika ausgewählt aus Bromiden und Chloriden der Kationen Tiotropium, Oxitropium, Flutropium, Ipratropium, Glycopyrronium und Trospium.

10. Pharmazeutischen Formulierung nach Anspruch 9, **gekennzeichnet durch** einen Gehalt an Tiotropiumbromid.

11. Pharmazeutischen Formulierung nach Anspruch 10, **gekennzeichnet durch** einen Gehalt an kristallinem Tiotropiumbromid Monohydrats.

## Claims

1. Enantiomerically pure compounds of formula 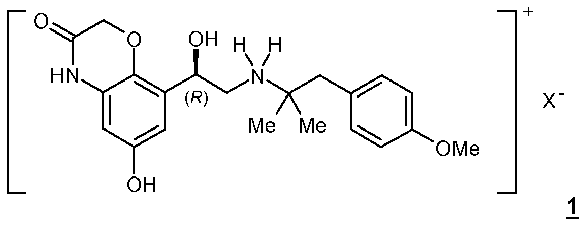 wherein the compounds are present in an enantiomeric purity of at least 85%ee and X-denotes chloride, optionally in the form of the tautomers, mixtures of the tautomers, hydrates or solvates thereof.

2. Enantiomerically pure compounds of formula **1** according to claim 1, **characterised in that** they are in crystalline form, optionally in the form of their crystalline tautomers, crystalline hydrates or crystalline solvates.

3. Enantiomerically pure compounds of formula 1 according to one of claims 1 or 2, the X-ray powder diagrams of which, recorded using a Bruker D8 Advanced with an LSD (= location sensitive detector) CuKα - radiation, λ = 1.5418 A, 30 kV, 40 mA, have the following lattice plane distances measured in A: d = 5.92 A; 5.81 A; 5.51 A; 5.10 Å; 4.65 A; 4.50 Å; 4.15 A and 4.00 Å.

4. Enantiomerically pure compounds of general formula **1** according to one of claims 1 to 3 as medicaments.

5. Enantiomerically pure compounds of general formula **1** according to one of claims 1 to 3 as medicaments for the treatment of respiratory complaints.

6. Use of enantiomerically pure compounds of general formula **1** according to one of claims 1 to 3 for preparing a medicament for the treatment of respiratory complaints.

7. Pharmaceutical formulation, **characterised in that** it contains a compound of formula **1** according to one of claims 1 to 3.

8. Pharmaceutical formulation according to claim 7, **characterised in that** it contains a compound of formula **1** according to one of claims 1 to 3 and one or more compounds which are selected from among the anticholinergics, PDEIV inhibitors, steroids, LTD4 antagonists and EGFR inhibitors.

9. Pharmaceutical formulation according to claim 8, **characterised in that** it contains one or more anticholinergics selected from among bromides and chlorides of the cations tiotropium, oxitropium, flutropium, ipratropium, glycopyrronium and trospium.

10. Pharmaceutical formulation according to claim 9, **characterised in that** it contains tiotropium bromide.

11. Pharmaceutical formulation according to claim 10, **characterised in that** it contains crystalline tiotropium bromide monohydrate.

## Revendications

1. Composés énantiomériquement purs de formule 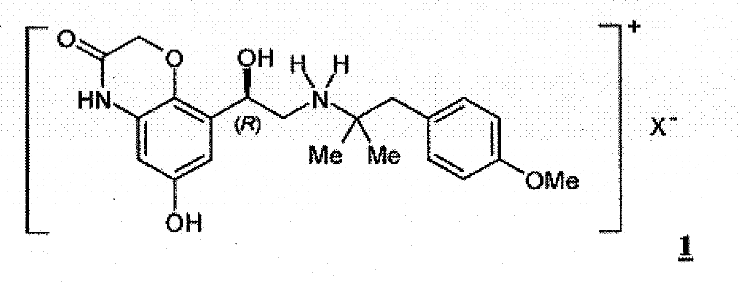 dans laquelle les composés se présentent en une pureté énantiomérique d'au moins 85 % ee et X⁻ représente un chlorure, éventuellement sous la forme d'un tautomère, de mélanges de tautomères, d'hydrates ou de solvates.

2. Composés énantiomériquement purs de formule 1 selon la revendication 1, **caractérisés en ce qu'**ils se présentent sous forme cristalline, éventuellement sous la forme de leurs tautomères cristallins, d'hydrates cristallins ou de solvates cristallins.

3. Composés énantiomériquement purs de formule générale 1 selon l'une des revendications 1 ou 2, dont le diagramme de diffraction de rayons X sur poudre enregistré au moyen d'un dispositif Bruker D8 Advanced avec un détecteur sensible à la position (PSD) de rayonnement CuK_{α}, λ = 1,5418 Å, 30 kV, 40 mA présente les écarts de réseau suivants en Å : d = 5,92 Å ; 5,81 Å ; 5,51 Å ; 5,10 Å ; 4,65 Å ; 4,50 Å ; 4,15 Å et 4,00 Å.

4. Composés énantiomériquement purs de formule générale 1 selon l'une des revendications 1 à 3, comme médicament.

5. Composés énantiomériquement purs de formule générale 1 selon l'une des revendications 1 à 3, comme médicament pour le traitement des maladies des voies respiratoires.

6. Utilisation de composés énantiomériquement purs de formule générale 1 selon l'une des revendications 1 à 3, pour la production d'un médicament pour le traitement de maladies des voies respiratoires.

7. Formulation pharmaceutique, **caractérisée par** une teneur en un composé de formule 1 selon l'une des revendications 1 à 3.

8. Formulation pharmaceutique selon la revendication 7, **caractérisée par** une teneur en un composé de formule 1 selon l'une des revendications 1 à 3 et un ou plusieurs composés qui sont choisis dans les classes des anticholinergiques, des inhibiteurs de PDEIV, des stéroïdes, des antagonistes de LTD4 et des inhibiteurs de l'EGFR.

9. Formulation pharmaceutique selon la revendication 8, **caractérisée par** une teneur en un ou plusieurs anticholinergiques choisis parmi les bromures et chlorures des cations tiotropium, oxitropium, flutropium, ipratropium, glycopyrronium et trospium.

10. Formulation pharmaceutique selon la revendication 9, **caractérisée par** une teneur en bromure de tiotropium.

11. Formulation pharmaceutique selon la revendication 10, **caractérisée par** une teneur en bromure de tiotropium monohydraté cristallin.
